# EUROPEAN PATENT SPECIFICATION

(11) **EP 3 139 906 B1**
(45) Date of publication and mention of the grant of the patent: **10.04.2019**
(21) Application number: 15722994.9
(22) Date of filing: 07.05.2015
(51) Int. Cl.: A61K 31/137, A61K 9/20

(54) **A SLOW-RELEASE PHARMACEUTICAL FORMULATION**
PHARMAZEUTISCHE FORMULIERUNG MIT LANGSAMER WIRKSTOFFABGABE
FORMULATION PHARMACEUTIQUE À LIBÉRATION LENTE

(30) Priority: 09.05.2014 EP 14167733
(43) Date of publication of application: 15.03.2017
(73) Proprietor: G.L. Pharma GmbH, 8502 Lannach (AT); Alfred E. Tiefenbacher GmbH & Co. KG, 22767 Hamburg (DE)
(72) Inventor: FLEMMING, Jens, 22143 Hamburg (DE); WAGNER, Harald, 8010 Graz (AT); GSÖLL, Martina, 8010 Graz (AT)
(74) Representative: Sonn & Partner Patentanwälte
(86) International application number: PCT/EP2015/060079
(87) International publication number: WO 2015/169904

(56) References cited:
- EP-B1- 1 439 829
- WO-A2-02/067651

## Description

(1R,2R)-3-(3-dimethylamino-1-ethyl-2-methyl-propyl)-phenol, which is also known as tapentadol (CAS no. 175591-23-8) is a synthetic, centrally acting analgesic effective in the treatment of pain.

Regarding its mechanism of action, it has been known that tapentadol exhibits a dual mechanism in that it acts on the one hand as a µ-opioid receptor agonist and on the other hand as a noradrenaline transporter inhibitor. In humans, the affinity of tapentadol to the recombinantly produced µ-opioid receptor is 18-times less than that of morphine. However, clinical studies have shown the pain-alleviating action of tapentadol to be only two to three times less than that of morphine. The only slightly reduced analgesic efficacy with a simultaneously 18-times reduced affinity to the recombinant µ-opioid receptor indicates that the noradrenaline transporter inhibiting property of tapentadol also contributes to its analgesic efficacy. Consequently, it may be assumed that tapentadol has a similar analgesic efficacy to that of pure µ-opioid receptor agonists but has fewer of the side effects associated with the µ-opioid receptor. The compound can be used in the form of its free base or as a salt or solvate. The production of the free tapentadol base and its hydrochloride salt are known for example from EP 0 693 475 A.

Conventional formulations for oral administration of a 3-(3-dimethylamino-1-ethyl-2-methyl-propyl)-phenol compound, such as tapentadol, usually lead to a rapid release of the active substance in the gastrointestinal tract, thereby leading to a rather rapid onset of its analgesic action. Subsequently, naturally also a rather rapid reduction in the action is observed. In order to achieve an effective analgesic action over a prolonged period of time, i.e. to ensure an adequately high concentration of the active substance in the patient's blood plasma, it would therefore be necessary to administer the pharmaceutical composition comprising said active substance at relatively short time intervals. However, the need for frequent dosing may lead to errors in administration and to undesirable variations in the concentration of the compound in the blood plasma which could be detrimental to patient compliance and the therapeutic benefit, particularly when treating chronically painful conditions.

If in the following reference is made to a "slow release", this should also include sustained release and prolonged release. Delayed release can be obtained when combining a formulation according to the present invention showing slow release with an appropriate coating.

In order to overcome the above mentioned disadvantages of conventional formulations, EP 1 439 829 A suggests providing a delayed-release pharmaceutical composition suitable for oral administration comprising 3-(3-dimethylamino-1-ethyl-2-methyl-propyl)-phenol or its hydrogen chloride salt, in which retardation is accomplished by means of a matrix, a coating or in a release system based on osmotic action, wherein the matrix, the coating or the release system based on osmotic action contains 1 to 80% by weight of one or more pharmaceutically acceptable hydrophilic or hydrophobic polymers as matrix forming agents. Such a pharmaceutical formulation is disclosed to achieve slow release of 3-(3-dimethylamino-1-ethyl-2-methyl-propyl)phenol and it is disclosed to be particularly surprising that such a formulation not only ensures long-lasting therapeutic efficacy over a relatively long period (at least 12 hours) due to the slow release of the active ingredient, but at the same time allows the active ingredient to start flowing rapidly in the plasma when the pharmaceutical composition is first administered, leading to a rapid onset of pain relief in the patient. The formulation according to EP 1 439 829 A is said to combine properties of a formulation with immediate release of active ingredient, namely rapid pain relief due to adequately high concentration of active ingredient just after administration of the pharmaceutical composition, with properties of a formulation having slow release, namely long-lasting analgesic action due to maintenance of an adequately high level of active ingredient over a prolonged time. By taking the analgesic in the formulation according to EP 1 439 829 A, the patient should be able to effectively combat his pain acutely and, at the same time, treat it effectively over a prolonged period without further measures and merely by regular administration at 12 (or 24) hourly intervals.

WO 02/067651 A2 relates to salts of 3-(3- dimethylamino-1-ethyl-2-methyl-propyl)-phenol in hydrophobic delayed release matrix tablets, particular preferably of mono- or diglycerides of C₁₂-C₃₀ fatty acids or their mixtures.

There is still a need for alternative administration forms comprising a 3-(3-dimethylamino-1-ethyl-2-methyl-propyl)-phenol compound such as tapentadol, by which a controlled, slow release of the active substance can be achieved. Preferably, such alternative administration forms provide for the same benefits as the ones according to EP 1 439 829 A, while avoiding the use of hydrophilic or hydrophobic polymers as matrix forming agents.

Lipid excipients are already widely used in slow release applications and can be processed by standard techniques such as direct compression and wet granulation or using melt methods. Compritol® as put onto the market by Gattefossé, a French privately-owned company based in Saint-Priest (Lyon, France), for instance, is a glyceryl behenate with low HLB and high melting point (70°C) commonly used as a release retarding agent in matrix tablets. To be more precise, Compritol 888 ATO is a mixture of mono- (12 - 18%), di- (52%) and triglycerides (28 - 32%) of behenic acid (C₂₁H₄₃COOH). Behenic acid (also docosanoic acid) is a carboxylic acid, in appearance it consists of white to cream colour crystals or powder with a melting point of 80 °C and boiling point of 306 °C. The drug release mechanism from Compritol® 888 matrices is diffusion dominated and independent of the processing technique, the drug dissolves and diffuses out of the matrix following water penetration into the dosage form. The importance of selecting the right diluent for Compritol® 888 matrix SR tablet formulations has already been shown, it also has already been disclosed how such a diluent could be used to modulate the drug release profile.

Reproducible slow release matrix systems rely on the use of a minimum concentration of excipient to create an infinite matrix network which entraps the drug and prevents its immediate release. This minimum excipient concentration is referred to as the "percolation threshold" and has to be considered when designing a slow release matrix system. The percolation threshold concept is well-described for hydrophilic polymer matrices, with 20 - 30% excipient content being cited. Studies concerning Compritol® 888 ATO (glyceryl dibehenate NF), a lipid matrix former frequently used to sustain drug release also have been published and it has been disclosed that Compritol® 888 ATO forms a release retarding matrix for freely water soluble drugs and controls release by diffusion.

For instance, slow-release levodopa mini-tablets (3 & 4mm) have already been manufactured and it is known that the release rate of levodopa from such slow-release mini-tablets may be modified by altering the amount of Compritol® 888 in the formulation and/or mini-tablet size, so that the desired release profile can be tailored to suit the clinical need.

Also nicotinic acid (niacin) containing slow release tablets have been manufactured by wet granulation as a simple and efficient process to obtain high dose niacin slow release lipid matrix tablets. A study has been published which describes the development of slow-release niacin tablets with Compritol® 888 using a wet granulation process. It also has already been shown that the addition of PVP improves the quality of tablets with limited effect on drug release. High dose lipid matrix tablets delivered slow release comparable to an existing authorized medicine. The ratio of drug to excipient enables tablet height and size to be considered as part of the overall dosage form design.

Precirol®, as put onto the market by Gattefossé, FR, is a glyceryl distearate, more precisely a mixture of mono-, di- und triglycerides of palmitic- and stearic acid. Precirol® is used within the European Union for human pharmaceutical products and veterinary products excluding food producing animals. It can be used to form lipid matrices for modified release tablets by direct compression, it provides lubrication for capsule filling, it is suitable for use in melt processing techniques and can also be used for hard gelatine capsule molding.

Aim of the present invention is the development of a slow release matrix tablet comprising tapentadol or a pharmaceutically acceptable salt thereof, which tablet can also easily be obtained by using melt methods and which tablet shows release rates in principle similar to the release rates of the formulations according to EP 1 439 829 A in general and to the release rates of the tapentadol containing product Palexia® in particular.

It has now surprisingly been found that this aim can be reached by a slow-release pharmaceutical formulation containing 3-(3-dimethylamino-1-ethyl-2-methyl-propyl)phenol or a pharmaceutically acceptable salt thereof as active ingredient in a slow release matrix, wherein the matrix contains between 15 and 50 wt.-% of mono-, di- and triglycerides of saturated fatty acids with a chain length between 16 and 22 carbon atoms and a mixture of such mono-, di- und triglycerides, respectively, as pharmaceutically acceptable matrix forming agents, is free of polymers as matrix forming agents and has the following release rate in vitro, measured by the Ph. Eur. Paddle Method at 100 rpm in a buffer (to Ph. Eur.) at a pH of 6.8 at 37 °C and detected using a UV spectrometer:
3 to 35% by weight 3-(3-dimethylamino-1-ethyl-2-methyl-propyl)phenol released after 0.5 hours,
5 to 50% by weight 3-(3-dimethylamino-1-ethyl-2-methyl-propyl)phenol released after 1 hour,
10 to 75% by weight 3-(3-dimethylamino-1-ethyl-2-methyl-propyl)phenol released after 2 hours,
15 to 82% by weight 3-(3-dimethylamino-1-ethyl-2-methyl-propyl)phenol released after 3 hours,
30 to 97% by weight 3-(3-dimethylamino-1-ethyl-2-methyl-propyl)phenol released after 6 hours,
more than 50% by weight 3-(3-dimethylamino-1-ethyl-2-methyl-propyl)phenol released after 12 hours,
more than 70% by weight 3-(3-dimethylamino-1-ethyl-2-methyl-propyl)phenol released after 18 hours, and
more than 80% by weight 3-(3-dimethylamino-1-ethyl-2-methyl-propyl)phenol released after 24 hours.

According to a preferred embodiment of the present invention, the slow-release pharmaceutical formulation has the following release rate in vitro, measured by the Ph. Eur. Paddle Method at 100 rpm in a buffer (to Ph. Eur.) at a pH of 6.8 at 37 °C and detected using a UV spectrometer:
5 to 23% by weight 3-(3-dimethylamino-1-ethyl-2-methyl-propyl)phenol released after 0.5 hours,
14 to 35% by weight 3-(3-dimethylamino-1-ethyl-2-methyl-propyl)phenol released after 1 hour,
25 to 47% by weight 3-(3-dimethylamino-1-ethyl-2-methyl-propyl)phenol released after 2 hours,
36 to 57% by weight 3-(3-dimethylamino-1-ethyl-2-methyl-propyl)phenol released after 3 hours,
59 to 80% by weight 3-(3-dimethylamino-1-ethyl-2-methyl-propyl)phenol released after 6 hours,
more than 75% by weight 3-(3-dimethylamino-1-ethyl-2-methyl-propyl)phenol released after 12 hours,
more than 80% by weight 3-(3-dimethylamino-1-ethyl-2-methyl-propyl)phenol released after 18 hours, and
more than 90% by weight 3-(3-dimethylamino-1-ethyl-2-methyl-propyl)phenol released after 24 hours.

Compritol 888 ATO, being a mixture of 12 - 18 wt.-% mono-, 52 wt.-% di- and 28 -32 wt.-% triglycerides of behenic acid, can be used in such a slow-release pharmaceutical formulation containing 3-(3-dimethylamino-1-ethyl-2-methyl-propyl)phenol or a pharmaceutically acceptable salt thereof as active ingredient, and Compritol 888 ATO can also be the only pharmaceutically acceptable matrix forming agent in such a formulation.

In an especially preferred embodiment of the present invention, the slow-release pharmaceutical formulation has the following release rate in vitro, measured by the Ph. Eur. Paddle Method at 100 rpm in a buffer (to Ph. Eur.) at a pH of 6.8 at 37 °C and detected using a UV spectrometer:
9 to 17% by weight 3-(3-dimethylamino-1-ethyl-2-methyl-propyl)phenol released after 0.5 hours,
21 to 27% by weight 3-(3-dimethylamino-1-ethyl-2-methyl-propyl)phenol released after 1 hour,
33 to 41% by weight 3-(3-dimethylamino-1-ethyl-2-methyl-propyl)phenol released after 2 hours,
41 to 51% by weight 3-(3-dimethylamino-1-ethyl-2-methyl-propyl)phenol released after 3 hours,
62 to 77% by weight 3-(3-dimethylamino-1-ethyl-2-methyl-propyl)phenol released after 6 hours,
more than 80% by weight 3-(3-dimethylamino-1-ethyl-2-methyl-propyl)phenol released after 12 hours,
more than 85% by weight 3-(3-dimethylamino-1-ethyl-2-methyl-propyl)phenol released after 18 hours, and
more than 90% by weight 3-(3-dimethylamino-1-ethyl-2-methyl-propyl)phenol released after 24 hours.

The percentages above are given based on 100% by weight active ingredient.

By substituting the matrix polymers according to EP 1 439 829 A with mono-, di- und triglycerides of saturated fatty acids with a chain length between 16 and 22 carbon atoms it is possible to obtain a slow release pharmaceutical formulation which is largely equivalent with products according to EP 1 439 829 A and/or the tapentadol containing product Palexia® as put onto the market by the patentee of EP 1 439 829 A. In connection therewith it is particularly noteworthy that in paragraph [0012] of EP 1 439 829 A fatty acid esters are mentioned as possible polymers to be used for building up the matrix of EP 1 439 829 A. It is known to a person skilled in the art that only esters of unsaturated or polyunsaturated fatty acids can undergo polymerisation, an example for which are drying or curing oils. The "drying", hardening, or, more properly, curing of these oils is the result of autoxidation and subsequent crosslinking. The process starts with oxygen inserting into carbon-hydrogen (C-H) bonds adjacent to one of the double bonds within the unsaturated fatty acid, the hydroperoxides thus formed are then susceptible to crosslinking reactions wherein bonds form between neighboring fatty acid chains, resulting in a polymer network. The polymerization results in stable films. Diene-containing fatty acid derivatives, such as those derived from linoleic acid, are especially prone to oxidation/polymerisation because they generate pentadienyl radicals. Monounsaturated fatty acids, such as oleic acid, are slower to undergo oxidation/polymerisation because the allylic radical intermediates are less stable.

Non-"drying" compounds as used according to the present invention, however, comprise long, spaghetti-like strands of hydrocarbon molecules, which interlace and compact but do not form covalent bonds in the manner of drying oils, i.e. they do not polymerise and thus cannot form polymers. Such compounds are re-dissoluble in an appropriate solvent whereas esters of unsaturated or polyunsaturated fatty acids undergo oxidation/polymerisation, are therefore considered cured and not soluble in solvents. Furthermore, while drying oils consist of glycerol triesters of fatty acids, these esters are characterized by high levels of polyunsaturated fatty acids, especially alpha-linolenic acid, which is also reflected in the iodine number of such compounds, which iodine number is an indicator of the number of double bonds in such compounds. Compounds with an iodine number greater than 130 are considered drying, those with an iodine number of 115-130 are semi-drying, and those with an iodine number of less than 115 are non-drying. Preferably, the compounds used according to the present invention have an iodine number of less than 50, even more preferred of less than 10, so that it is ensured that they cannot polymerise.

Furthermore, it has been found that it is of advantage if a heat treatment is carried out after direct compression of the tablets. Such a heat treatment may consist in exposing the formed tablets to elevated temperature for a certain period of time, for instance by exposing them to a temperature of below or close to or slightly above the melting point of the matrix, preferably below 75°C and more preferably at about 60°C in a dry oven during a period of time sufficient to harden the tablet, preferably during about 1 hour or less, more preferably during about 30 minutes.

According to a preferred embodiment of the present application the formulation is characterised in that the matrix contains a mixture of mono-, di- und triglycerides of saturated fatty acids with a chain length between 16 and 22 carbon atoms.

Preferably, the formulation according to the present invention is characterised in that the matrix contains a mixture of mono-, di- und triglycerides of behenic acid, palmitic acid and stearic acid.

It is also advantageous if the formulation according to the present invention is characterised in that the matrix contains a mixture of 12 - 18 wt.-% mono-, 52 wt.-% di- and 28 -32 wt.-% triglycerides of behenic acid, together with mono-, di- und triglycerides of palmitic acid and stearic acid. Compritol 888 ATO (glycerol behenate) and Precirol ATO 5 (glycerol distearate), both supplied by Gattefossé, can advantageously be used in the formulation according to the present invention, with Compritol 888 ATO being a mixture of 12 - 18 wt.-% mono-, 52 wt.-% di- and 28 -32 wt.-% triglycerides of behenic acid and Precirol ATO 5 being a mixture of mono-, di- und triglycerides of palmitic acid and stearic acid. According to their technical data sheet, both Compritol and Precirol show an iodine number of <= 3.0 gl2/100 g, meaning that they cannot polymerise. Furthermore, Precirol is easily soluble in chloroform and methylene chloride, while Compritol is soluble in chloroform and methylene chloride under heating conditions. The solubility of these two mixtures of fatty acid glycerides is further proof that they are no polymers, since polymers of fatty acid esters are always insoluble.

According to a further embodiment, the formulation according to the present invention is characterised in that the content of the slow release active ingredient is between 0,5 and 85 wt.-% and the content of pharmaceutically acceptable matrix forming agent is between 15 and 30 wt.-%, preferably between 20 and 30 wt.-%, and in particular about 25 wt.-%.

Still according to a further embodiment, the above-mentioned formulation is characterised in that the matrix is formed with a weight ratio of the glycerides of behenic acid to the glycerides of palmitic acid and stearic acid of between 2:1 and 1:1, preferably of about 3:2.

The formulation according to the present invention may reach the peak plasma level of the active ingredient in vivo after 2 to 10 hours, in particular after 3.5 to 6 hours.

According to yet another embodiment of the present invention, that formulation is characterised in that it contains (+)-(1S,2S)-3-(3-dimethylamino-1-ethyl-2-methyl-propyl)phenol or a pharmaceutically acceptable salt thereof.

Advantageously, the formulation according to the present invention is characterised in that it contains (-)-(1R,2R)-3-(3-dimethylamino-1-ethyl-2-methyl-propyl)phenol or a pharmaceutically acceptable salt thereof. According to a further embodiment of the present invention, a tablet for twice-daily oral administration of 3-(3-dimethylamino-1-ethyl-2-methyl-propyl)phenol, containing a pharmaceutical formulation according to the present invention is provided.

The retarding agent used in the following examples is based on a matrix system containing Compritol 888 ATO (glycerol behenate) and Precirol ATO 5 (glycerol distearate), both supplied by Gattefossé. To reinforce the strength and robustness of the formulation, a heat treatment is carried out after direct compression of the tablets. Therefore, the tablets are exposed to 60°C for 30 min in a dry oven.

In order to sustain the release of the highly soluble API, several formulations containing different excipients were tested with general regard to processability, hardness, friability and dissolution behavior.

The formulation development and the basic formulation (VCH 073/14) is based on a 50 mg dosage strength and consists of the API Tapentadol HCl, Compritol and Precirol as retarding agents, lactose and calcium hydrogen phosphate as fillers and magnesium stearate as lubricant (see Table 1). The tablets were prepared on a conventional tablet press (Korsch EK0) with a diameter of 8 mm and a crown radius of 8 mm.

**Table 1 - Basic formulation, 50 mg dosage strength**

| Material | mg/tbl | %/tbl |
|---|---|---|
| Tapentadol HCl | 58,24 | 20 |
| Compritol 888 ATO | 43,68 | 15 |
| Precirol ATO5 | 29,12 | 10 |
| Calcium Hydrogen Phosphate, anhydrous | 104,83 | 36 |
| Lactose (SuperTab 30GR) | 52,41 | 18 |
| Magnesium Stearate | 2,91 | 1 |
| | 291,19 | 100 |

Based on this formulation, several trial batches were manufactured and analyzed in matters of their dissolution behavior (in comparison to the competitor product), hardness and friability. Dissolution tests were performed under Phar. Eur. Conditions: pH 6.8, 1000 mL, 100 rpm, paddle without sinkers.

Variations on the basic formulation VCH 073/14 (Table 1):

### 1) Microcrystalline cellulose (VCH 062/14) (comparative example not according to the invention)

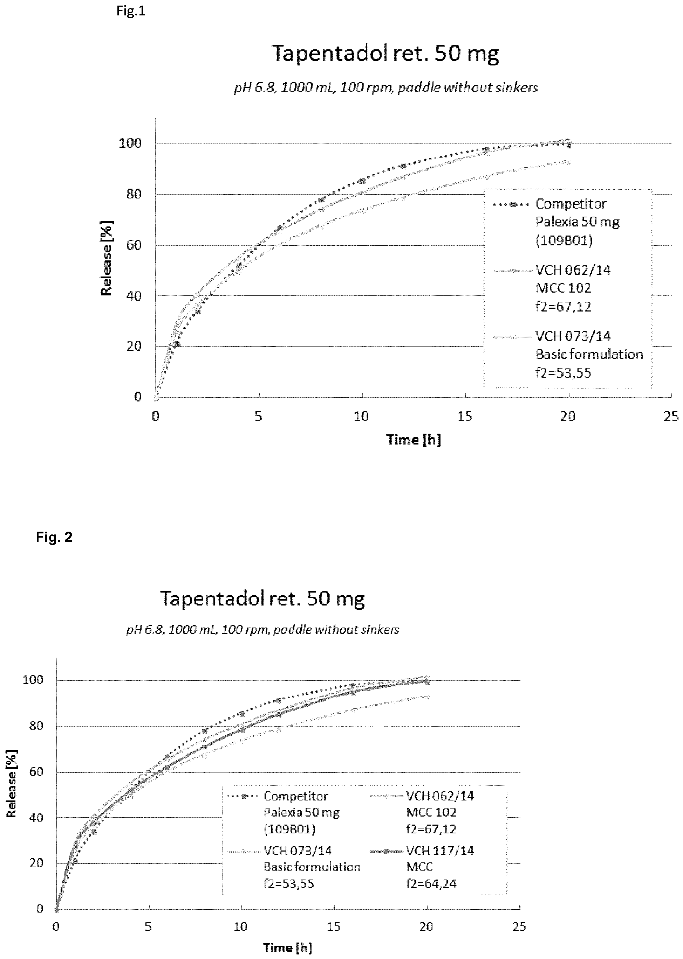

In this formulation the lactose content is completely replaced by microcrystalline cellulose (MCC 102). This resulted in a faster API release compared to the basic formulation and also higher hardness of the tablets. (Fig. 1)

| | *VCH 073*/*14* | | *VCH 062*/*14* | |
|---|---|---|---|---|
| **Material** | **mg/tbl** | **%/tbl** | **mg/tbl** | **%/tbl** |
| Tapentadol HCl | 58,24 | 20 | 58,24 | 20 |
| Compritol 888 ATO | 43,68 | 15 | 43,68 | 15 |
| Precirol ATO5 | 29,12 | 10 | 29,12 | 10 |
| CaHPO₄, anhydr. | 104,83 | 36 | 104,83 | 36 |
| Lactose (SuperTab 30GR) | 52,41 | 18 | - | - |
| **MCC 102** | **-** | **-** | **52,41** | **18** |
| Magnesium Stearate | 2,91 | 1 | 2,91 | 1 |
| **Total** | **291,19** | **100** | **291,19** | **100** |
| Compaction force [kN] | 27 | | 27 | |
| Hardness[N] | 63 | | 77 | |
| Friability [%] | 0,20 | | 0,13 | |

### 2) Increase of microcrystalline cellulose (VCH 117/14) (comparative example not according to the invention)

Compared to formulation trial VCH 062/14, where lactose was completely replaced by MCC, additional 25% of the content of CaHPO₄ are replaced by MCC.

This led to a very slight decrease of API release rate and an increase of tablet hardness. Fluctuations within press capacity and tablet weight were observed during manufacturing. (Fig. 2)

| | *VCH 073*/*14* | | *VCH 117*/*14* | |
|---|---|---|---|---|
| **Material** | **mg/tbl** | **%/tbl** | **mg/tbl** | **%/tbl** |
| Tapentadol HCl | 58,24 | 20 | 58,24 | 20 |
| Compritol 888 ATO | 43,68 | 15 | 43,68 | 15 |
| Precirol ATO5 | 29,12 | 10 | 29,12 | 10 |
| **CaHPO₄, anhydr.** | **104,83** | **36** | **78,62** | **36** |
| Lactose (SuperTab 30GR) | 52,41 | 18 | - | - |
| **MCC 102** | **-** | **-** | **78,62** | **18** |
| Magnesium Stearate | 2,91 | 1 | 2,91 | 1 |
| **Total** | **291,19** | **100** | **291,19** | **100** |
| Compaction force [kN] | 27 | | 27 | |
| Hardness[N] | 63 | | 79 | |
| Friability [%] | 0,20 | | 0,15 | |

### 3) Further increase of microcrystalline cellulose (VCH 132/14) (comparative example not according to the invention)

Compared to formulation trial VCH 117/14, where 25% of the content of CaHPO₄ were replaced by MCC, in this trial 75% of CaHPO₄ are replaced by MCC.

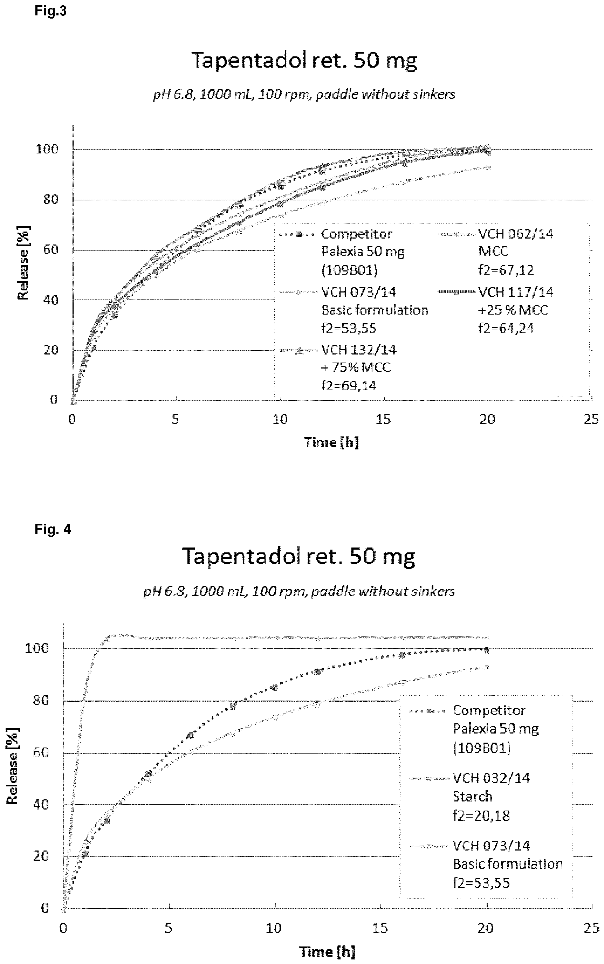

Overall API-release rate increased, but the high amount of MCC and lack of CaHPO₄, respectively, led to capping of the tablets! (Fig. 3)

| | *VCH 117*/*14* | | *VCH 132*/*14* | |
|---|---|---|---|---|
| **Material** | **mg/tbl** | **%/tbl** | **mg/tbl** | **%/tbl** |
| Tapentadol HCl | 58,24 | 20 | 58,24 | 20 |
| Compritol 888 ATO | 43,68 | 15 | 43,68 | 15 |
| Precirol ATO5 | 29,12 | 10 | 29,12 | 10 |
| **CaHPO₄, anhydr.** | **78,62** | **36** | **26,21** | **9** |
| Lactose (SuperTab 30GR) | - | - | - | - |
| **MCC 102** | **78,62** | **18** | **131,03** | **45** |
| Magnesium Stearate | 2,91 | 1 | 2,91 | 1 |
| **Total** | **291,19** | **100** | **291,19** | **100** |
| Compaction force [kN] | 27 | | 27 | |
| Hardness[N] | 79 | | 70 | |
| Friability [%] | 0,15 | | 0,17 | |

### 4) Starch, Starcap 1500 (VCH 032/14) (comparative example not according to the invention)

Altering the lactose content to starch (Starcap1500) leads to an immediate drug release which demonstrates that starch acts as disintegrant at such high concentrations. The hardness of the tablets remains almost unaffected. (Fig. 4)

| | *VCH 073*/*14* | | *VCH 032*/*14* | |
|---|---|---|---|---|
| **Material** | **mg/tbl** | **%/tbl** | **mg/tbl** | **%/tbl** |
| Tapentadol HCl | 58,24 | 20 | 58,24 | 20 |
| Compritol 888 ATO | 43,68 | 15 | 43,68 | 15 |
| Precirol ATO5 | 29,12 | 10 | 29,12 | 10 |
| CaHPO₄, anhydr. | 104,83 | 36 | 104,83 | 36 |
| Lactose (SuperTab 30GR) | 52,41 | 18 | - | - |
| **Starch, Starcap1500** | **-** | **-** | **52,41** | **18** |
| Magnesium Stearate | 2,91 | 1 | 2,91 | 1 |
| **Total** | **291,19** | **100** | **291,19** | **100** |
| Compaction force [kN] | 27 | | 27 | |
| Hardness[N] | 63 | | 66 | |
| Friability [%] | 0,20 | | 0,14 | |

### 5) Neusilin, Aluminium Magnesium Silicate (VCH 350/13)

In this formulation a small amount of Neusilin (Aluminium Magnesium Silicate) is added as a binder and disintegrant. To keep the tablet weight constant, little parts of lactose and calcium hydrogen phosphate are removed.

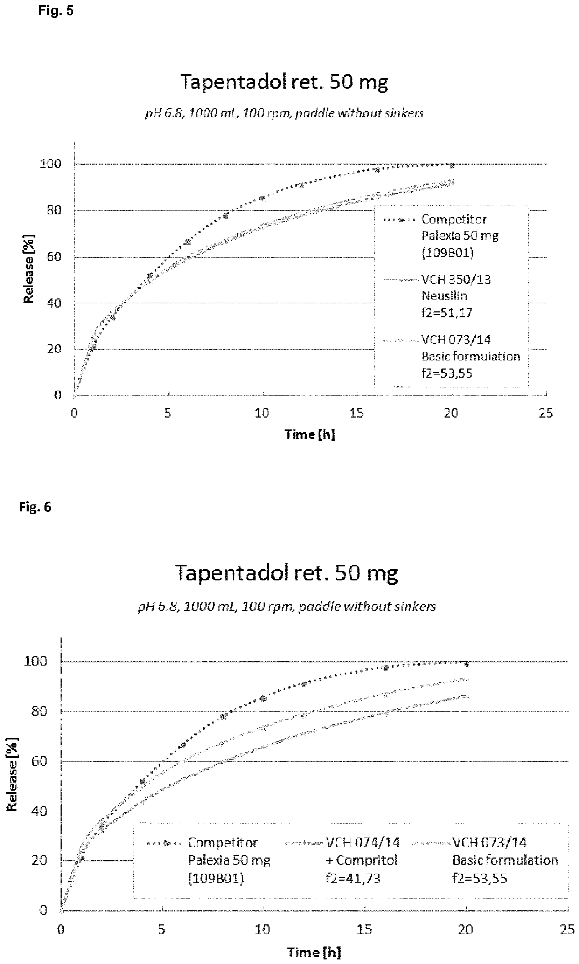

This change led to almost no differences in the dissolution profile compared to the basic formulation. Likewise, tablet hardness was not affected. (Fig. 5)

| | *VCH 073*/*14* | | *VCH 350*/*13* | |
|---|---|---|---|---|
| **Material** | **mg/tbl** | **%/tbl** | **mg/tbl** | **%/tbl** |
| Tapentadol HCl | 58,24 | 20 | 58,24 | 20 |
| Compritol 888 ATO | 43,68 | 15 | 43,68 | 15 |
| Precirol ATO5 | 29,12 | 10 | 29,12 | 10 |
| CaHPO₄, anhydr. | 104,83 | 36 | 101,91 | 35 |
| Lactose (SuperTab 30GR) | 52,41 | 18 | 46,59 | 16 |
| **Neusilin** | **-** | **-** | **8,74** | **3** |
| Magnesium Stearate | 2,91 | 1 | 2,91 | 1 |
| **Total** | **291,19** | **100** | **291,19** | **100** |
| Compaction force [kN] | 27 | | 27 | |
| Hardness[N] | 63 | | 64 | |
| Friability [%] | 0,20 | | 0,16 | |

### 6) Increase of Compritol and total weight (VCH 074/14)

In order to investigate the effect of Compritol on the formulation, its amount and thus, the total weight of the tablet is increased by ^{∼}6%.

As a result, the speed of API-release decreased over the entire dissolution time. Again, this change within the formulation did not influence tablet hardness. (Fig. 6)

| | *VCH 073*/*14* | | *VCH 074*/*14* | |
|---|---|---|---|---|
| **Material** | **mg/tbl** | **%/tbl** | **mg/tbl** | **%/tbl** |
| Tapentadol HCl | 58,24 | 20 | 58,24 | 18,8 |
| **Compritol 888 ATO** | **43,68** | **15** | **62,49** | **20,2** |
| Precirol ATO5 | 29,12 | 10 | 29,12 | 9,4 |
| CaHPO₄, anhydr. | 104,83 | 36 | 104,83 | 33,8 |
| Lactose (SuperTab 30GR) | 52,41 | 18 | 52,41 | 16,9 |
| Magnesium Stearate | 2,91 | 1 | 2,91 | 0,9 |
| **Total** | **291,19** | **100** | **310,00** | **100** |
| Compaction force [kN] | 27 | | 27 | |
| Hardness[N] | 63 | | 64 | |
| Friability [%] | 0,20 | | 0,14 | |

### 7) Decrease of Compritol and total weight (VCH 102/14)

In comparison to VCH 073/14 and VCH 074/14 the tablet weight of the basic formulation is decreased by reducing the amount of Compritol.

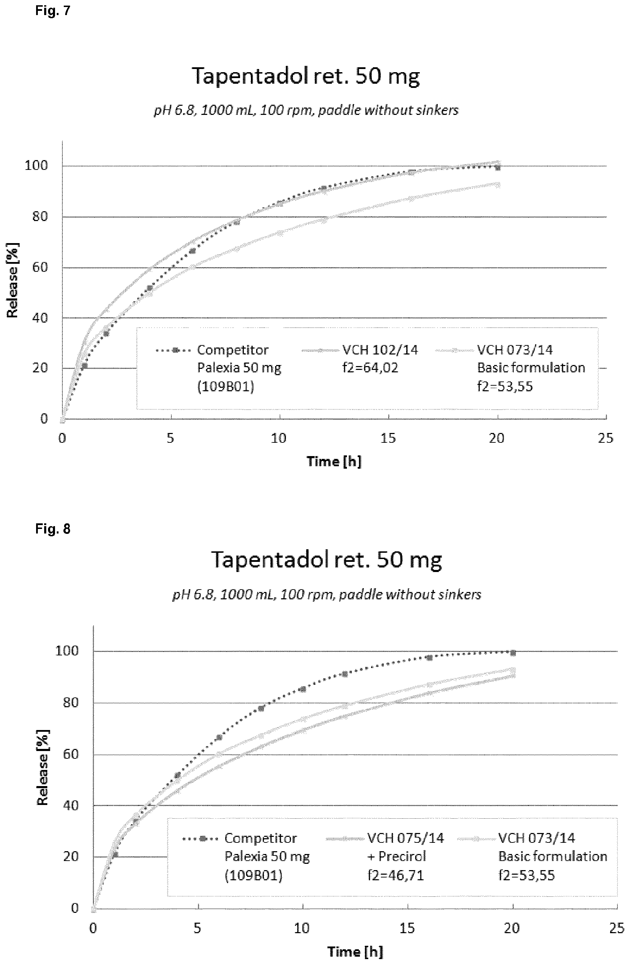

This increased the speed of API release, but there was no influence on tablet hardness. (Fig. 7)

| | *VCH 073*/*14* | | *VCH 102*/*14* | |
|---|---|---|---|---|
| **Material** | **mg/tbl** | **%/tbl** | **mg/tbl** | **%/tbl** |
| Tapentadol HCl | 58,24 | 20 | 58,24 | 21,4 |
| **Compritol 888 ATO** | **43,68** | **15** | **24,86** | **9,1** |
| Precirol ATO5 | 29,12 | 10 | 29,12 | 10,7 |
| CaHPO₄, anhydr. | 104,83 | 36 | 104,83 | 38,5 |
| Lactose (SuperTab 30GR) | 52,41 | 18 | 52,41 | 19,2 |
| Magnesium Stearate | 2,91 | 1 | 2,91 | 1,1 |
| **Total** | **291,19** | **100** | **272,37** | **100** |
| Compaction force [kN] | 27 | | 27 | |
| Hardness[N] | 63 | | 60 | |
| Friability [%] | 0,20 | | 0,28 | |

### 8) Increase of Precirol and total weight (VCH 075/14)

According to Compritol, the formulation is repeated for Precirol in the same way: its impact on tablet properties is investigated by increasing the content of Precirol and the total tablet weight.

In this case, the speed of API-release also decreased, but to a much lower extent compared to the Compritol-formulation. Nevertheless, tablet hardness remained unchanged. (Fig. 8)

| | *VCH 073*/*14* | | *VCH 075*/*14* | |
|---|---|---|---|---|
| **Material** | **mg/tbl** | **%/tbl** | **mg/tbl** | **%/tbl** |
| Tapentadol HCl | 58,24 | 20 | 58,24 | 18,8 |
| Compritol 888 ATO | 43,68 | 15 | 43,68 | 14,1 |
| **Precirol ATO5** | **29,12** | **10** | **47,93** | **15,5** |
| CaHPO₄, anhydr. | 104,83 | 36 | 104,83 | 33,8 |
| Lactose (SuperTab 30GR) | 52,41 | 18 | 52,41 | 16,9 |
| Magnesium Stearate | 2,91 | 1 | 2,91 | 0,9 |
| **Total** | **291,19** | **100** | **310,00** | **100** |
| Compaction force [kN] | 27 | | 27 | |
| Hardness[N] | 63 | | 62 | |
| Friability [%] | 0,20 | | 0,17 | |

### 9) Increase of calcium hydrogen phosphate and total weight (VCH 063/14)

The same procedure is conducted with calcium hydrogen phosphate: increasing its content and total tablet weight in order to explore the effect on dissolution and hardness.

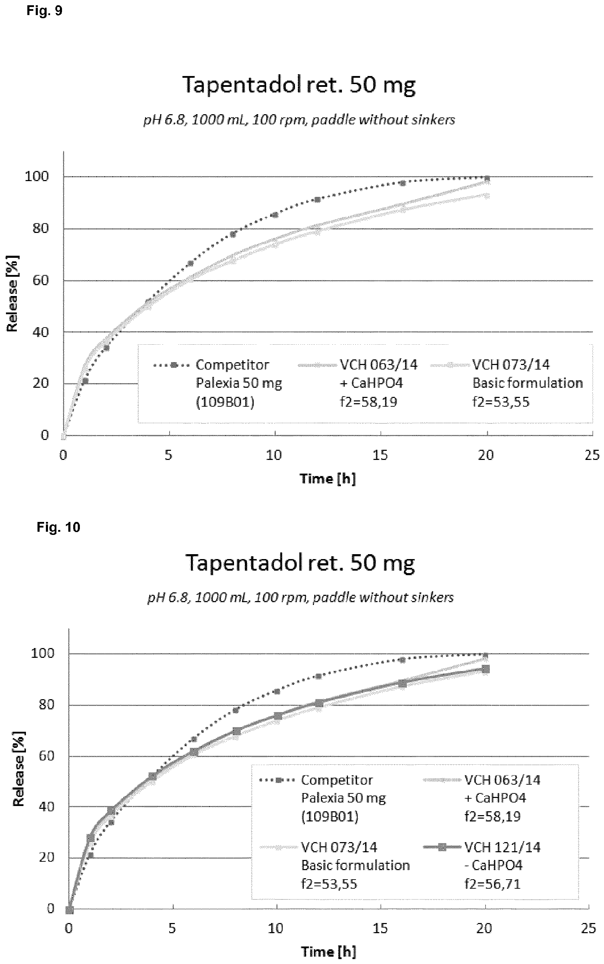

Raising the amount of calcium hydrogen phosphate did barely influence the retarding characteristics of the formulation, but led to slightly harder tablets. This suggests that calcium hydrogen phosphate only acts as filler and does not contribute to the retarding mechanism of the formulation. (Fig. 9)

| | *VCH 073*/*14* | | *VCH 063*/*14* | |
|---|---|---|---|---|
| **Material** | **mg/tbl** | **%/tbl** | **mg/tbl** | **%/tbl** |
| Tapentadol HCl | 58,24 | 20 | 58,24 | 18,8 |
| Compritol 888 ATO | 43,68 | 15 | 43,68 | 14,1 |
| Precirol ATO5 | 29,12 | 10 | 29,12 | 9,4 |
| **CaHPO₄, anhydr.** | **104,83** | **36** | **123,64** | **39,9** |
| Lactose (SuperTab 30GR) | 52,41 | 18 | 52,41 | 16,9 |
| Magnesium Stearate | 2,91 | 1 | 2,91 | 0,9 |
| **Total** | **291,19** | **100** | **310,00** | **100** |
| Compaction force [kN] | 27 | | 27 | |
| Hardness[N] | 63 | | 70 | |
| Friability [%] | 0,20 | | 0,16 | |

### 10) Decrease of calcium hydrogen phosphate and total weight (VCH 121/14)

In dependance on increasing the amount of CaHPO₄ and the total tablet weight, a formulation trial is performed by decreasing these parameters proportionally.

Almost any difference was observed regarding the dissolution profile, which indicates that CaHPO₄ does not contribute to the retardation system. In contrast, tablet hardness significantly decreased by reducing the amount of CaHPO₄. (Fig. 10)

| | *VCH 073*/*14* | | *VCH 121*/*14* | |
|---|---|---|---|---|
| **Material** | **mg/tbl** | **%/tbl** | **mg/tbl** | **%/tbl** |
| Tapentadol HCl | 58,24 | 20 | 58,24 | 21,4 |
| Compritol 888 ATO | 43,68 | 15 | 43,68 | 16,0 |
| Precirol ATO5 | 29,12 | 10 | 29,12 | 10,7 |
| **CaHPO₄, anhydr.** | **104,83** | **36** | **86,01** | **31,6** |
| Lactose (SuperTab 30GR) | 52,41 | 18 | 52,41 | 19,2 |
| Magnesium Stearate | 2,91 | 1 | 2,91 | 1,1 |
| **Total** | **291,19** | **100** | **272,37** | **100** |
| Compaction force [kN] | 27 | | 27 | |
| Hardness[N] | 63 | | 53 | |
| Friability [%] | 0,20 | | 0,20 | |

### 11) Increasing the total tablet weight of the basic formulation (VCH 101/14)

In order to examine the influence of tablet weight on dissolution behavior particularly, the basic formulation is proportionally increased by total weight up to 310 mg.

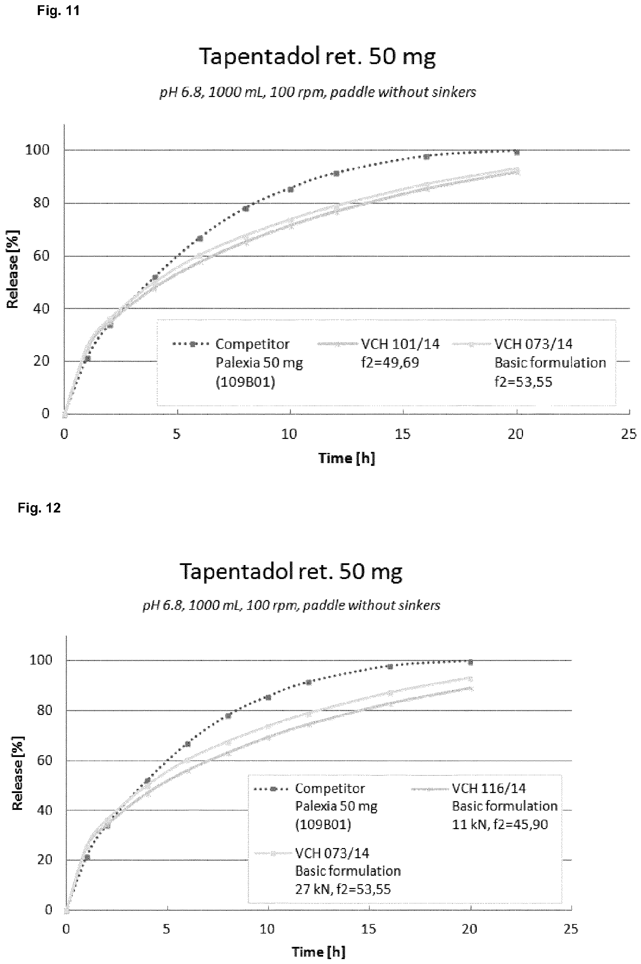

As expected, the release speed of the API decreased, but only to a very low extent. Again, tablet hardness remained unaffected. (Fig. 11)

| | *VCH 073*/*14* | | *VCH 101*/*14* | |
|---|---|---|---|---|
| **Material** | **mg/tbl** | **%/tbl** | **mg/tbl** | **%/tbl** |
| Tapentadol HCl | 58,24 | 20 | 58,24 | 18,8 |
| Compritol 888 ATO | 43,68 | 15 | 47,20 | 15,2 |
| Precirol ATO5 | 29,12 | 10 | 31,47 | 10,2 |
| CaHPO₄, anhydr. | 104,83 | 36 | 113,29 | 36,6 |
| Lactose (SuperTab 30GR) | 52,41 | 18 | 56,65 | 18,3 |
| Magnesium Stearate | 2,91 | 1 | 3,15 | 1,0 |
| **Total** | **291,19** | **100** | **310,00** | **100** |
| Compaction force [kN] | 27 | | 27 | |
| Hardness[N] | 63 | | 64 | |
| Friability [%] | 0,20 | | 0,15 | |

### 12) Replication of the basic formulation by altering compression force (VCH 116/14)

For investigating the influence of compression force on tablet hardness and dissolution behavior, the basic formulation is repeated with varying compression forces.

One can see a low increase of tablet hardness with increasing compression force. Considering the dissolution profile, low compression force led to a decrease of API-release speed. (Fig. 12)

| **Compression force [kN]** | **Hardness [N]** | **Friability [%]** |
|---|---|---|
| 11 | 55 | 0,12 |
| 20 | 59 | 0,08 |
| 24 | 60 | 0,23 |
| 27 | 63 | 0,20 |

### 13) Replication of the basic formulation by altering tablet dimensions (VCH 131/14)

For investigating the influence of tablet dimension on hardness and dissolution behavior, the basic formulation is repeated with altering the punch size from 8 mm to 12 x 5 mm.

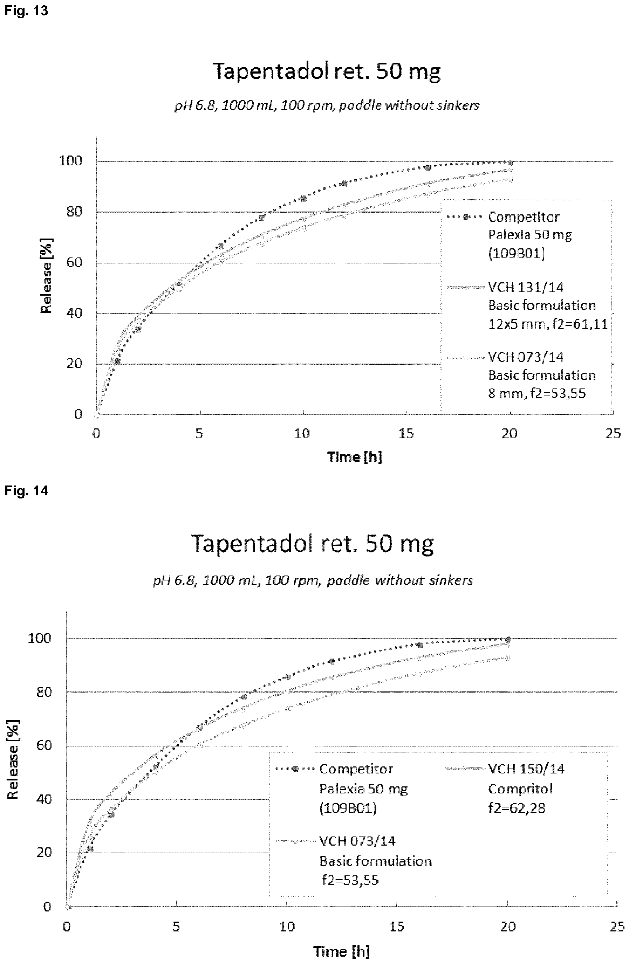

This led to a slight increase of overall API release. Due to lower compaction force of the 12 x 5 mm punch, tablet hardness decreased. (Fig. 13)

| | **8 mm** | **12 x 5 mm** |
|---|---|---|
| **Compaction force [kN]** | 27 | 17,1 |
| **Hardness [N]** | 63 | 46 |
| **Friability [%]** | 0,20 | 0,15 |

### 14) Replacing Precirol by Compritol

To investigate the impact of compritol and mainly the lack of Precirol on the formulation and dissolution behavior, Precirol is eliminated and replaced by Compritol. In this way, the interaction of both excipients can be identified. There is to be said, that high fluctuations were noted within compression force, which complicated the manufacturing process.

An increase of API-release speed and a decrease of tablet hardness were observed. A higher tablet hardness could be obtained by replacing lactose by MCC. (Fig. 14)

| | *VCH 073*/*14* | | *VCH 150*/*14* | |
|---|---|---|---|---|
| **Material** | **mg/tbl** | **%/tbl** | **mg/tbl** | **%/tbl** |
| Tapentadol HCl | 58,24 | 20 | 58,24 | 20 |
| **Compritol 888 ATO** | 43,68 | 15 | **72,80** | **25** |
| **Precirol ATO5** | 29,12 | 10 | - | - |
| CaHPO₄, anhydr. | 104,83 | 36 | 104,83 | 36 |
| Lactose (SuperTab 30GR) | 52,41 | 18 | 52,41 | 18 |
| Magnesium Stearate | 2,91 | 1 | 2,91 | 1 |
| **Total** | **291,19** | **100** | **291,19** | **100** |
| Compaction force [kN] | 27 | | ^{∼}27 | |
| Hardness [N] | 63 | | 51 | |
| Friability [%] | 0,20 | | 0,21 | |

### 15) Replacing Compritol by Precirol

According to Compritol, the influence of solely Precirol on the formulation is investigated by replacing the total content of Compritol by Precirol. There is to be said, that high fluctuations were noted within compression force, which complicated the manufacturing process!

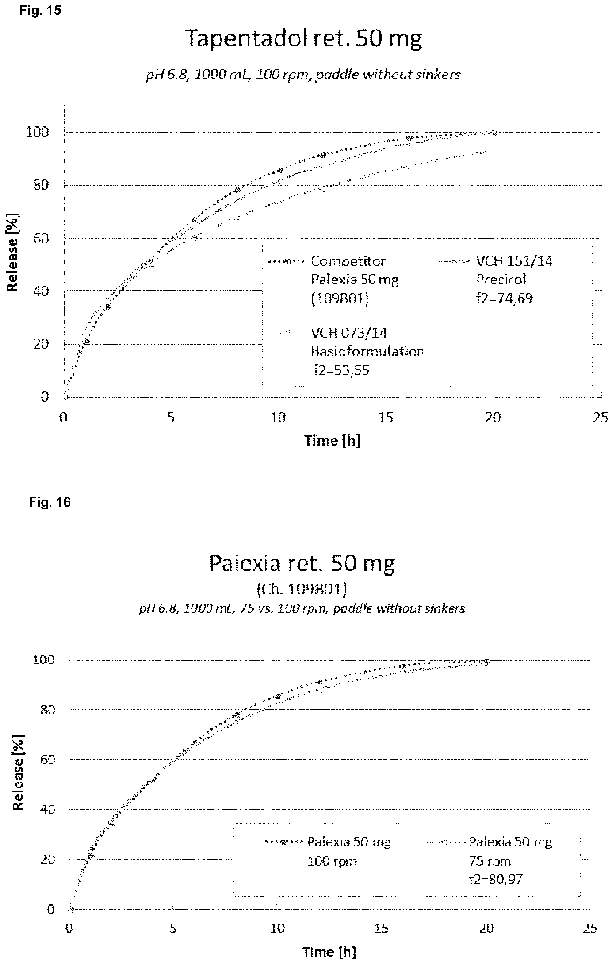

As a result, the API-release speed increased and tablet hardness decreased. (Fig. 15)

| | *VCH 073*/*14* | | *VCH 151*/*14* | |
|---|---|---|---|---|
| **Material** | **mg/tbl** | **%/tbl** | **mg/tbl** | **%/tbl** |
| Tapentadol HCl | 58,24 | 20 | 58,24 | 20 |
| **Compritol 888 ATO** | 43,68 | 15 | - | - |
| **Precirol ATO5** | 29,12 | 10 | **72,80** | **25** |
| CaHPO₄, anhydr. | 104,83 | 36 | 104,83 | 36 |
| Lactose (SuperTab 30GR) | 52,41 | 18 | 52,41 | 18 |
| Magnesium Stearate | 2,91 | 1 | 2,91 | 1 |
| **Total** | **291,19** | **100** | **291,19** | **100** |
| Compaction force [kN] | 27 | | ^{∼}27 | |
| Hardness[N] | 63 | | 55 | |
| Friability [%] | 0,20 | | 0,22 | |

### 16) Comparison Palexia 50 mg: 75 rpm vs. 100 rpm

For investigating the influence of rotational speed on API-release of the competitor product Palexia, dissolution tests are performed at 75 rpm and 100 rpm.

Almost any difference can be seen in the dissolution profile, which indicates that there is marginal impact of rotational speed on dissolution behavior. (Fig. 16)

### 17) Use of SMCC and lack of Precirol (VCH 283/14) (comparative example not according to the invention)

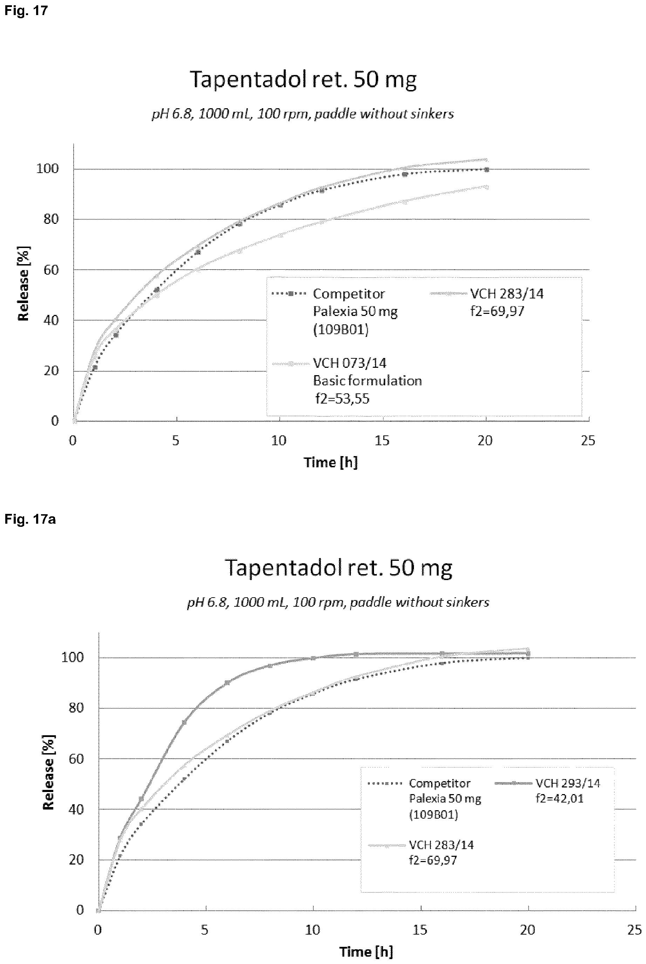

In this formulation the lactose content is replaced by silicified microcrystalline cellulose (SMCC). In addition, Precirol was completely removed and CaHPO₄ dihydrate was used instead of the anhydrous form. The curing step was performed by 60°C for 3 hours and the manufacturing process was a wet granulation. (Fig. 17)

| | *VCH 073*/*14* | | *VCH 283*/*14* | |
|---|---|---|---|---|
| **Material** | **mg/tbl** | **%/tbl** | **mg/tbl** | **%/tbl** |
| Tapentadol HCl | 58,24 | 20,00 | 58,24 | 19,41 |
| Compritol 888 ATO | 43,68 | 15,00 | 140,30 | 46,77 |
| Precirol ATO5 | 29,12 | 10,00 | - | |
| CaHPO₄, anhydr. | 104,83 | 36,00 | - | - |
| CaHPO₄, dihydrate | - | - | 29,16 | 9,72 |
| Lactose (SuperTab 30GR) | 52,41 | 18,00 | - | |
| MCC 102 | - | - | - | |
| SMCC | - | - | 69,30 | 23,10 |
| Magnesium Stearate | 2,91 | 1,00 | 3,00 | 1,00 |
| **Total** | **291,19** | **100,00** | **300,00** | **100,00** |
| Compaction force [kN] | 27 | | 27 | |
| Hardness [N] | 63 | | 70 | |
| Friability [%] | 0,20 | | 0,10 | |

### a) Elimination of Magnesium Stearate (based on VCH 283/14) (comparative example not according to the invention)

Compared to VCH 283/14 in this formulation Magnesium stearate was removed. This led to a decrease in hardness and increased speed of API release. (Fig. 17a)

| | ***VCH 283*/*14*** | | ***VCH 293*/*14*** | |
|---|---|---|---|---|
| **Material** | **mg/tbl** | **%/tbl** | **mg/tbl** | **%/tbl** |
| **Tapentadol HCl** | **58,24** | **19,41** | **58,24** | **19,61** |
| **Compritol 888 ATO** | **140,30** | **46,77** | **140,30** | **47,24** |
| **CaHPO₄, dihydrate** | **29,16** | **9,72** | **29,16** | **9,82** |
| **SMCC** | **69,30** | **23,10** | **69,30** | **2,33** |
| **Magnesium Stearate** | **3,00** | **1,00** | **-** | |
| **Total** | **300,00** | **100,00** | **297,00** | **100,00** |
| **Compaction force [kN]** | **27** | | **27** | |
| **Hardness [N]** | **70** | | **42** | |
| **Friability [%]** | **0,10** | | **0,00** | |

### 18) Change of manufacturing method to melt granulation

These trials were manufactured by melt granulation and the only ingredients were the API and Compritol.

### a) Variation of Compritol content

For investigating the influence of the amount of Compritol on the dissolution behavior, various weight ratios of the two compounds were tested in a formulation (see table below).

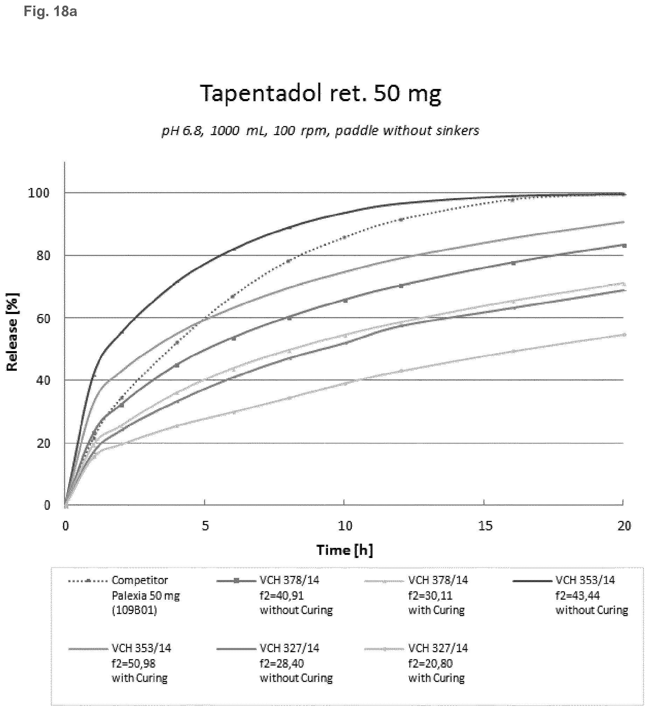

As expected, an increase in Compritol led to decreased dissolution speed. Besides, the additional curing step retarded the dissolution of the API. (Fig. 18a)

| | *VCH 353*/*14* | | *378*/*14* | | *327*/*14* | |
|---|---|---|---|---|---|---|
| **Material** | **mg/tbl** | **%/tbl** | **mg/tbl** | **%/tbl** | **mg/tbl** | **%/tbl** |
| Tapentadol HCl | 58,24 | 50,00 | 58,24 | 35,00 | 58,24 | 25,00 |
| Compritol 888 ATO | 58,24 | 50,00 | 108,16 | 65,00 | 174,72 | 75,00 |
| **Total** | **116,48** | **100,00** | **166,40** | **100,00** | **232,96** | **100,00** |
| Compaction force [kN] | 26 | | 23 | | 28 | |
| Hardness [N] | 26 | | 27 | | 35 | |
| Friability [%] | 0,02 | | 0,08 | | 0,03 | |
| Crown radius [mm] | 7 | | 7 | | 8 | |

### b) Addition of SMCC and CaHPO₄ (comparative example not according to the invention)

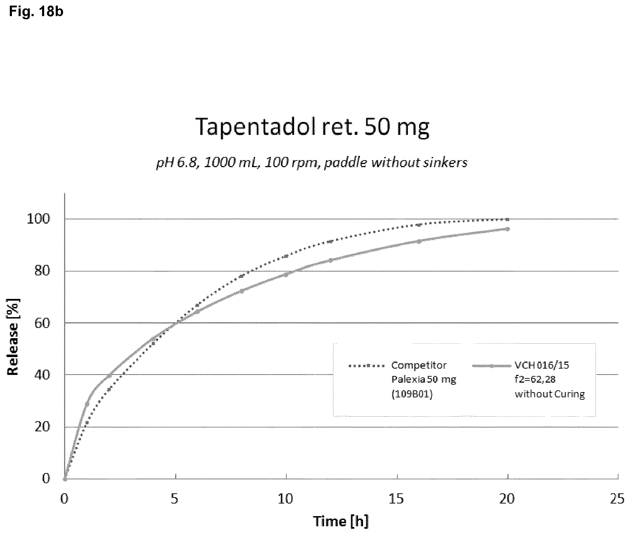

In order to increase the hardness of the tablets, SMCC and CaHPO₄ were added in this formulation. Due to these excipients, a slight increase in tablet hardness was observed. (Fig. 18b)

| | *VCH 016*/*15* | |
|---|---|---|
| **Material** | **mg/tbl** | **%/tbl** |
| Tapentadol HCl | 58,24 | 33,28 |
| Compritol 888 ATO | 87,36 | 49,92 |
| CaHPO₄, dihydrate | 11,90 | 6,80 |
| SMCC | 17,50 | 10,00 |
| **Total** | **175,00** | **100,00** |
| Compaction force [kN] | 19 | |
| Hardness [N] | 44 | |
| Friability [%] | 0,03 | |
| Crown radius [mm] | 6,5 | |

## Claims

1. A slow-release pharmaceutical formulation containing 3-(3-dimethylamino-1-ethyl-2-methylpropyl)phenol or a pharmaceutically acceptable salt thereof as active ingredient in a slow release matrix, wherein the matrix contains between 15 and 50 wt.-% of mono-, di- and triglycerides of saturated fatty acids with a chain length between 16 and 22 carbon atoms and a mixture of such mono- di- and triglycerides, respectively, as pharmaceutically acceptable matrix forming agents, the matrix being free of polymers as matrix forming agents and having the following release rate in vitro, measured by the Ph. Eur. Paddle Method at 100 rpm in a buffer (to Ph. Eur.) at a pH of 6.8 at 37° C and detected using a UV spectrometer:
3 to 35 % by weight (based on 100% by weight active ingredient) 3-(3-dimethylamino-1-ethyl-2-methylpropyl)phenol released after 0.5 hours,
5 to 50% by weight 3-(3-dimethylamino-1-ethyl-2-methyl-propyl)phenol released after 1 hour,
10 to 75% by weight 3-(3-dimethylamino-1-ethyl-2-methyl-propyl)phenol released after 2 hours,
15 to 82% by weight 3-(3-dimethylamino-1-ethyl-2-methyl-propyl)phenol released after 3 hours,
30 to 97% by weight 3-(3-dimethylamino-1-ethyl-2-methyl-propyl)phenol released after 6 hours,
more than 50% by weight 3-(3-dimethylamino-1-ethyl-2-methyl-propyl)phenol released after 12 hours,
more than 70% by weight 3-(3-dimethylamino-1-ethyl-2-methyl-propyl)phenol released after 18 hours, and
more than 80% by weight 3-(3-dimethylamino-1-ethyl-2-methyl-propyl)phenol released after 24 hours.

2. Formulation according to claim 1, **characterised in that** the matrix contains a mixture of mono-, di- und triglycerides of saturated fatty acids with a chain length between 16 and 22 carbon atoms.

3. Formulation according to one of claims 1 or 2, **characterised in that** the matrix contains a mixture of mono-, di- und triglycerides of behenic acid, palmitic acid and stearic acid.

4. Formulation according to one of claims 1 to 3, **characterised in that** the matrix contains a mixture of 12 - 18 wt.-% mono-, 52 wt.-% di- and 28 -32 wt.-% triglycerides of behenic acid, together with mono-, di- und triglycerides of palmitic acid and stearic acid.

5. Formulation according to one of claims 1 to 4, **characterised in that** the content of the slow release active ingredient is between 0.5 and 85 wt.-% and the content of pharmaceutically acceptable matrix forming agent is between 15 and 30 wt.-%, preferably between 20 and 30 wt.-%, and in particular about 25 wt.-%.

6. Formulation according to claim 5, **characterised in that** the matrix is formed with a weight ratio of the glycerides of behenic acid to the glycerides of palmitic acid and stearic acid of between 2:1 and 1:1, preferably of about 3:2.

7. Formulation according to one of claims 1 to 6, **characterised in that** it contains (+)-(1S,2S)-3-(3-dimethylamino-1-ethyl-2-methyl-propyl)phenol or a pharmaceutically acceptable salt thereof.

8. Formulation according to one of the claims 1 to 7, **characterised in that** it contains (-)-(1R,2R)-3-(3-dimethylamino-1-ethyl-2-methyl-propyl)phenol or a pharmaceutically acceptable salt thereof.

9. Tablet for twice-daily oral administration of 3-(3-dimethylamino-1-ethyl-2-methyl-propyl)phenol, containing a pharmaceutical formulation according to one of claims 1 to 8.

## Patentansprüche

1. Pharmazeutische Formulierung mit langsamer Wirkstofffreisetzung, enthaltend 3-(3-Dimethylamino-1-ethyl-2-methyl-propyl) phenol oder ein pharmazeutisch verträgliches Salz von diesem als wirksamen Bestandteil in einer Matrix mit langsamer Wirkstofffreisetzung, wobei die Matrix zwischen 15 und 50 Gew.-% an Mono-, Diund Triglyceriden von gesättigten Fettsäuren mit einer Kettenlänge von zwischen 16 und 22 Kohlenstoffatomen bzw. ein Gemisch aus derartigen Mono-, Di- und Triglyceriden als pharmazeutisch verträgliche Matrixbildner enthält, wobei die Matrix keine Polymere als Matrixbildner enthält und über die folgende *in vitro* Freisetzungsrate verfügt, laut Messung gemäß dem Flügelrührer-Verfahren (*Paddle Method,* siehe Ph. Eur.) bei 100 rpm in einem Puffer (gemäß Ph. Eur.) bei einem pH-Wert von 6,8 und 37° C sowie Detektion unter Verwendung eines UV-Spektrometers:
3 bis 35 Gew.-% (basierend auf 100 Gew.-% des wirksamen Bestandteils) an 3-(3-Dimethylamino-1-ethyl-2-methyl-propyl)phenol freigesetzt nach 0,5 Stunden,
5 bis 50 Gew.-% an 3-(3-Dimethylamino-1-ethyl-2-methyl-propyl) phenol freigesetzt nach 1 Stunde,
10 bis 75 Gew.-% an 3-(3-Dimethylamino-1-ethyl-2-methyl-propyl)phenol freigesetzt nach 2 Stunden,
15 bis 82 Gew.-% an 3-(3-Dimethylamino-1-ethyl-2-methyl-propyl)phenol freigesetzt nach 3 Stunden,
30 bis 97 Gew.-% an 3-(3-Dimethylamino-1-ethyl-2-methyl-propyl)phenol freigesetzt nach 6 Stunden,
mehr als 50 Gew.-% an 3-(3-Dimethylamino-1-ethyl-2-methyl-propyl)phenol freigesetzt nach 12 Stunden,
mehr als 70 Gew.-% an 3-(3-Dimethylamino-1-ethyl-2-methyl-propyl)phenol freigesetzt nach 18 Stunden, und
mehr als 80 Gew.-% an 3-(3-Dimethylamino-1-ethyl-2-methyl-propyl)phenol freigesetzt nach 24 Stunden.

2. Formulierung nach Anspruch 1, **dadurch gekennzeichnet, dass** die Matrix ein Gemisch aus Mono-, Di- und Triglyceriden von gesättigten Fettsäuren mit einer Kettenlänge von zwischen 16 und 22 Kohlenstoffatomen enthält.

3. Formulierung nach einem der Ansprüche 1 oder 2, **dadurch gekennzeichnet, dass** die Matrix ein Gemisch aus Mono-, Di- und Triglyceriden von Behensäure, Palmitinsäure und Stearinsäure enthält.

4. Formulierung nach einem der Ansprüche 1 bis 3, **dadurch gekennzeichnet, dass** die Matrix ein Gemisch aus 12 bis 18 Gew.-% an Mono-, 52 Gew.-% an Di- und 28 bis 32 Gew.-% an Triglyceriden von Behensäure zusammen mit Mono-, Di- und Triglyceriden von Palmitinsäure und Stearinsäure enthält.

5. Formulierung nach einem der Ansprüche 1 bis 4, **dadurch gekennzeichnet, dass** der Gehalt des zur langsamen Freisetzung vorgesehenen wirksamen Bestandteils zwischen 0,5 und 85 Gew.-% beträgt und der Gehalt des pharmazeutisch verträglichen Matrixbildners zwischen 15 und 30 Gew.-%, bevorzugt zwischen 20 und 30 Gew.- % und insbesondere etwa 25 Gew.-% beträgt.

6. Formulierung nach Anspruch 5, **dadurch gekennzeichnet, dass** die Matrix mit einem Gewichtsverhältnis von zwischen 2:1 und 1:1, bevorzugt von etwa 3:2, der Glyceride von Behensäure zu den Glyceriden von Palmitinsäure und Stearinsäure gebildet wird.

7. Formulierung nach einem der Ansprüche 1 bis 6, **dadurch gekennzeichnet, dass** die Formulierung (+)-(1S,2S)-3-(3-Dimethylamino-1-ethyl-2-methyl-propyl)phenol oder ein pharmazeutisch verträgliches Salz von diesem enthält.

8. Formulierung nach einem der Ansprüche 1 bis 7, **dadurch gekennzeichnet, dass** die Formulierung (-)-(1R,2R)-3-(3-Dimethylamino-1-ethyl-2-methyl-propyl)phenol oder ein pharmazeutisch verträgliches Salz von diesem enthält.

9. Tablette zur zwei Mal täglichen oralen Verabreichung von 3-(3-Dimethylamino-1-ethyl-2-methyl-propyl)phenol, enthaltend eine pharmazeutische Formulierung nach einem der Ansprüche 1 bis 8.

## Revendications

1. Formulation pharmaceutique à libération lente contenant, en tant que principe actif, du 3-(3-diméthylamino-1-éthyl-2-méthylpropyl)phénol ou un sel pharmaceutiquement acceptable de celui-ci, dans une matrice à libération lente, la matrice contenant entre 15 et 50 % en poids de mono-, di- et tri-glycérides d'acides gras saturés ayant une longueur de chaîne comprise entre 16 et 22 atomes de carbone et un mélange de tels mono-, di- et tri-glycérides, respectivement, en tant qu'agents de formation de matrice pharmaceutiquement acceptables, la matrice étant exempte de polymères servant d'agents de formation de matrice et ayant le taux de libération in vitro suivant, mesuré par la méthode à la palette de la Pharmacopée Européenne à 100 t/min dans un tampon (de la Pharmacopée Européenne), à un pH de 6,8, à 37°C, et détecté par utilisation d'un spectromètre UV :
3 à 35 % en poids (pour 100 % en poids de principe actif) de 3-(3-diméthylamino-1-éthyl-2-méthylpropyl)phénol libérés après 0,5 heure,
5 à 50 % en poids de 3-(3-diméthylamino-1-éthyl-2-méthylpropyl)phénol libérés après 1 heure,
10 à 75 % en poids de 3-(3-diméthylamino-1-éthyl-2-méthylpropyl)phénol libérés après 2 heures,
15 à 82 % en poids de 3-(3-diméthylamino-1-éthyl-2-méthylpropyl)phénol libérés après 3 heures,
30 à 97 % en poids de 3-(3-diméthylamino-1-éthyl-2-méthylpropyl)phénol libérés après 6 heures,
plus de 50 % en poids de 3-(3-diméthylamino-1-éthyl-2-méthylpropyl)phénol libérés après 12 heures,
plus de 70 % en poids de 3-(3-diméthylamino-1-éthyl-2-méthylpropyl)phénol libérés après 18 heures, et
plus de 80 % en poids de 3-(3-diméthylamino-1-éthyl-2-méthylpropyl)phénol libérés après 24 heures.

2. Formulation selon la revendication 1, **caractérisée en ce que** la matrice contient un mélange de mono-, di- et tri-glycérides d'acides gras saturés ayant une longueur de chaîne comprise entre 16 et 22 atomes de carbone.

3. Formulation selon l'une quelconque des revendications 1 et 2, **caractérisée en ce que** la matrice contient un mélange de mono-, di- et tri-glycérides d'acide béhénique, d'acide palmitique et d'acide stéarique.

4. Formulation selon l'une quelconque des revendications 1 à 3, **caractérisée en ce que** la matrice contient un mélange de 12 à 18 % en poids de mono-, 52 % en poids de di- et 28 à 32 % en poids de tri-glycérides d'acide béhénique, conjointement avec des mono-, di- et tri-glycérides d'acide palmitique et d'acide stéarique.

5. Formulation selon l'une quelconque des revendications 1 à 4, **caractérisée en ce que** la teneur en ledit principe actif à libération lente est comprise entre 0,5 et 85 % en poids, et la teneur en ledit agent de formation de matrice pharmaceutiquement acceptable est comprise entre 15 et 30 % en poids, de préférence entre 20 et 30 % en poids et en particulier est d'environ 25 % en poids.

6. Formulation selon la revendication 5, **caractérisée en ce que** la matrice est formée avec un rapport en poids des glycérides d'acide béhénique aux glycérides d'acide palmitique et d'acide stéarique compris entre 2/1 et 1/1, de préférence d'environ 3/2.

7. Formulation selon l'une quelconque des revendications 1 à 6, **caractérisée en ce qu'**elle contient du (+)-(1S,2S)-3-(3-diméthylamino-1-éthyl-2-méthylpropyl)phénol ou un sel pharmaceutiquement acceptable de celui-ci.

8. Formulation selon l'une quelconque des revendications 1 à 7, **caractérisée en ce qu'**elle contient du (-)-(1R,2R)-3-(3-diméthylamino-1-éthyl-2-méthylpropyl)phénol ou un sel pharmaceutiquement acceptable de celui-ci.

9. Comprimé pour une administration par voie orale deux fois par jour de 3-(3-diméthylamino-1-éthyl-2-méthylpropyl)phénol, contenant une formulation pharmaceutique selon l'une quelconque des revendications 1 à 8.
